# EUROPEAN PATENT SPECIFICATION

(11) **EP 4 335 372 B1**
(45) Date of publication and mention of the grant of the patent: **28.01.2026**
(21) Application number: 22804052.3
(22) Date of filing: 20.05.2022
(51) Int. Cl.: A61B 5/263, C23C 14/06, A61B 5/28

(54) **ECG ELECTRODE AND PREPARATION METHOD THEREFOR, AND ELECTRONIC DEVICE**
EKG-ELEKTRODE UND HERSTELLUNGSVERFAHREN DAFÜR SOWIE ELEKTRONISCHE VORRICHTUNG
ÉLECTRODE ECG ET SON PROCÉDÉ DE PRÉPARATION, ET DISPOSITIF ÉLECTRONIQUE

(30) Priority: 21.05.2021 CN 202110560688
(43) Date of publication of application: 13.03.2024
(73) Proprietor: Huawei Technologies Co., Ltd., Longgang Shenzhen, Guangdong 518129 (CN)
(72) Inventor: HUANG, Yujian, Shenzhen, Guangdong 518129 (CN); JIN, Qiu, Shenzhen, Guangdong 518129 (CN); CAI, Ming, Shenzhen, Guangdong 518129 (CN); LI, Longyu, Shenzhen, Guangdong 518129 (CN)
(74) Representative: Epping - Hermann - Fischer
(86) International application number: PCT/CN2022/094008
(87) International publication number: WO 2022/242732

(56) References cited:
- CN-A- 102 102 171
- CN-A- 103 199 279
- CN-A- 105 644 059
- CN-A- 106 282 918
- CN-A- 106 282 918
- CN-A- 107 881 466
- CN-A- 108 011 115
- CN-A- 108 400 177
- CN-A- 109 055 910
- CN-A- 113 991 134
- CN-U- 208 822 755
- JP-A- 2017 215 387
- ZHANG DI ET AL: "Amorphous carbon films doped with silver and chromium to achieve ultra-low interfacial electrical resistance and long-term durability in the application of proton exchange membrane fuel cells", CARBON, vol. 145, 20 January 2019 (2019-01-20), pages 333 - 344, XP085669921, ISSN: 0008-6223, DOI: 10.1016/J.CARBON.2019.01.050
- YI PEIYUN ET AL: "Carbon-based coatings for metallic bipolar plates used in proton exchange membrane fuel cells", INTERNATIONAL JOURNAL OF HYDROGEN ENERGY, vol. 44, no. 13, 14 February 2019 (2019-02-14), pages 6813 - 6843, XP085614385, ISSN: 0360-3199, DOI: 10.1016/J.IJHYDENE.2019.01.176
- YAN FANGYUAN ET AL: "Majorization of GLC properties by the introduction of silver nanowires as conductive framework for metal bipolar plates", APPLIED SURFACE SCIENCE, ELSEVIER, AMSTERDAM , NL, vol. 533, 10 August 2020 (2020-08-10), XP086304518, ISSN: 0169-4332, [retrieved on 20200810], DOI: 10.1016/J.APSUSC.2020.147493
- G�NTER SCHULTES ET AL: "Granular metal-carbon nanocomposites as piezoresistive sensor films - Part 1: Experimental results and morphology", JOURNAL OF SENSORS AND SENSOR SYSTEMS, 1 January 2018 (2018-01-01), Gottingen, pages 1 - 11, XP055508295, Retrieved from the Internet <URL:https://jsss.copernicus.org/articles/7/1/2018/> [retrieved on 20180919], DOI: 10.5194/jsss-7-1-2018

## Description

This disclosure claims priority to Chinese Patent Application No. 202110560688.9, filed with the China National Intellectual Property Administration on May 21, 2021 and entitled "ECG ELECTRODE, FABRICATION METHOD THEREOF, AND ELECTRONIC DEVICE".

### TECHNICAL FIELD

This disclosure relates to the field of material technologies, and more specifically, to an ECG electrode, a fabrication method thereof, and an electronic device.

### BACKGROUND

Currently, electronic devices such as a smartwatch and a smart band keep developing. These smart electronic devices usually include an ECG (Electrocardiography, electrocardiography) electrode. The ECG electrode is at a location that is on a housing of the smart electronic device and that is in contact with skin, and is configured to monitor the heart of a person (or referred to as a "monitored object") wearing the smart terminal device. Stainless steel or titanium alloy is usually used as a substrate material of the ECG electrode. Both stainless steel and titanium alloy have defects of low hardness and poor wear resistance. As a result, with long-term use of the electronic device, the ECG electrode may be worn out or even become invalid, to disable the electronic device to monitor an electrocardiogram any longer.

ZHANG DI ET AL discloses in "Amorphous carbon films doped with silver and chromium to achieve ultra-low interfacial electrical resistance and long-term durability in the application of proton exchange membrane fuel cells", XP085669921, that amorphous carbon (a-C) films exhibit properties that make them attractive for a wide range of applications in automotive components. Proton exchange membrane fuel cells (PEMFCs) assembled by metallic bipolar plates (BPPs) are promising power sources for new energy vehicles. Harsh environments of PEMFCs require preparing coatings on metallic BPPs to achieve high corrosion resistance and electrical conductivity, and a-C based coatings have the great potential. However, their low stability and high internal stress significantly damage their durability. This document aims to develop a fundamental understanding of the effect of doping Ag and Cr on the performance and durability of a-C based coatings. Experimental results revealed that a-C films doped with Ag and Cr (a-C:Ag:Cr) simultaneously achieve ultra-low interfacial contact resistance (ICR), low internal stresses, and higher stabilities. An atomistic model by molecular dynamics (MD) is developed to understand certain behaviors of a-C:Ag:Cr. The ultra-low ICR was mainly attributed to Ag nanoclusters embedded in a-C phases, low hardness, and a more graphite-like structure. The enhanced stability was attributed to denser structures and lower internal stresses. MD simulations suggested that the effect of doping Ag or Cr on the surface morphology and internal stress of a-C films may have different mechanisms.

### SUMMARY

This disclosure provides an ECG electrode. An outer surface of an electrode substrate of the ECG electrode is plated with a doped graphite-like carbon film, so that the ECG electrode has excellent conductivity, and also has high hardness and high wear resistance. In addition, this disclosure further provides a fabrication method for the ECG electrode and an electronic device using the ECG electrode.

The present invention is set out by the set of appended claims. In the following, parts of the description and drawing referring to implementations or examples, which are not covered by the claims are not presented as embodiments of the invention, but as illustrative examples useful for understanding the invention. The embodiments of the present invention are provided by the claims. According to a first aspect, an embodiment of this disclosure provides an ECG electrode, including an electrode substrate and a doped graphite-like carbon film located on an outer surface of the electrode substrate. The doped graphite-like carbon film includes graphite-like carbon, and first nano-crystals and second nano-crystals that are doped in the graphite-like carbon. The graphite-like carbon includes a carbon element. The graphite-like carbon has an amorphous carbon structure. The first nano-crystal includes a weak carbon-bonding element. The weak carbon-bonding element has a part that exists in a form of a metallic elementary substance. The first nano-crystal includes a columnar structure. The columnar structure extends in a thickness direction of the doped graphite-like carbon film. The second nano-crystal includes a strong carbon-bonding element. The strong carbon-bonding element bonds with the carbon element in the graphite-like carbon through a chemical bond. The second nano-crystals are distributed between adjacent and spaced columnar structures.

In the foregoing technical solution, the graphite-like carbon (graphite-like carbon, GLC for short) has advantages of high hardness and good wear resistance, but has poor conductivity in a thickness direction. The first nano-crystal of the columnar structure is embedded in the amorphous carbon structure of the graphite-like carbon, to enhance the conductivity of the graphite-like carbon in the thickness direction of the graphite-like carbon. However, in addition, introduction of the first nano-crystal reduces density and hardness inside the graphite-like carbon, so that the hardness and the wear resistance of the graphite-like carbon are reduced. The second nano-crystal is introduced in the amorphous carbon structure of the graphite-like carbon and the columnar structure of the first nano-crystal, to enhance strength and wear resistance of the doped graphite-like carbon film.

With reference to the first aspect, in a first possible implementation of the first aspect, the weak carbon-bonding element includes one or more of the following materials: Cu, Ag, Au, and Al. **In** the foregoing technical solution, the weak carbon-bonding element is difficult to chemically bond with the carbon element, and mainly exists in the form of the metallic elementary substance in the graphite-like carbon. The metals Cu, Ag, Au, and Al have excellent conductivity, so that the formed first nano-crystal is embedded in the amorphous carbon structure of the graphite-like carbon, to enhance the conductivity of the graphite-like carbon.

With reference to the first aspect or the first possible implementation of the first aspect, in a second possible implementation of the first aspect, the doped graphite-like carbon film includes a first surface facing the electrode substrate and a second surface opposite to the first surface. An atomic percentage of the weak carbon-bonding element gradually increases in a thickness direction from the first surface to the second surface. **In** the foregoing technical solution, as the atomic percentage of the weak carbon-bonding element increases, the first nano-crystal is precipitated in the columnar structure. The first nano-crystal of the columnar structure is also referred to as a columnar nano-crystal. The columnar nano-crystal including the weak carbon-bonding elements Cu, Ag, Au, and Al has excellent conductivity, and may form a longitudinal conductive channel in the graphite-like carbon, so that the conductivity of the graphite-like carbon in the thickness direction of the graphite-like carbon is enhanced, and high ECG signal quality is achieved.

With reference to the first aspect or any one of the first to the second possible implementations of the first aspect, in a third possible implementation of the first aspect, the doped graphite-like carbon film includes the first surface facing the electrode substrate and the second surface opposite to the first surface. A first nano-crystal close to the first surface is of a cluster structure. A first nano-crystal close to the second surface is of a columnar structure. **In** the foregoing technical solution, an atomic percentage of a weak carbon-bonding element close to the first surface is low, and a first nano-crystal formed from the weak carbon-bonding element is precipitated in a cluster structure. An atomic percentage of a weak carbon-bonding element closer to the second surface is greater, and a first nano-crystal formed from the weak carbon-bonding element is precipitated in a columnar structure.

With reference to the third possible implementation of the first aspect, in a fourth possible implementation of the first aspect, the second nano-crystals are distributed between a plurality of cluster structures and a plurality of columnar structures. In the foregoing technical solution, a distribution manner of the second nano-crystals and the first nano-crystals helps enhance bonding force inside the doped graphite-like carbon film. In addition, because the second nano-crystal is a hard carbide, embedding the second nano-crystals between the cluster structures and the columnar structures of the first nano-crystals helps better enhance the hardness of the entire doped graphite-like carbon film.

With reference to the first aspect or any one of the first to the fourth possible implementations of the first aspect, in a fifth possible implementation of the first aspect, the strong carbon-bonding element includes one or more of the following materials: Cr, W, and Ti. The strong carbon-bonding element bonds with the carbon element in the graphite-like carbon through the chemical bond, to generate second nano-crystals CrC, WC, and TiC. The foregoing three carbides are all hard carbides, and have advantages of high hardness and high wear resistance. The second nano-crystal is embedded in the amorphous carbon structure of the graphite-like carbon, to improve the hardness of the doped graphite-like carbon film and enhance the wear resistance of the doped graphite-like carbon film.

With reference to the first aspect or any one of the first to the fifth possible implementations of the first aspect, in a sixth possible implementation of the first aspect, the doped graphite-like carbon film includes the first surface facing the electrode substrate and the second surface opposite to the first surface. An atomic percentage of the strong carbon-bonding element gradually decreases in the thickness direction from the first surface to the second surface. In the foregoing technical solution, a great atomic percentage of the weak carbon-bonding element may be maintained close to the second surface, so that the doped graphite-like carbon film maintains high electrical signal quality close to the second surface. In addition, from the first surface to the second surface, the atomic percentage of the strong carbon-bonding element gradually decreases, and the atomic percentage of the weak carbon-bonding element gradually increases. Such a complementary design helps improve bonding force of the doped graphite-like carbon film.

With reference to the first aspect or any one of the first to the sixth possible implementations of the first aspect, in a seventh possible implementation of the first aspect, the ECG electrode further includes a transition layer, and the transition layer is located between the electrode substrate and the doped graphite-like carbon film. In the foregoing technical solution, atoms may be diffused between the transition layer and the electrode substrate and between the transition layer and the doped graphite-like carbon film, to improve bonding force between the electrode substrate and the doped graphite-like carbon film.

With reference to the seventh implementation of the first aspect, in an eighth possible implementation of the first aspect, the transition layer includes one or more of the following materials: Cr, W, and Ti. The transition layer is made of a same material as the strong carbon-bonding element in the doped graphite-like carbon film, to further improve bonding force between the transition layer and the doped graphite-like carbon film.

According to a second aspect, an embodiment of this disclosure further provides an electronic device, including a processor and the ECG electrode according to any one of the foregoing implementations. The ECG electrode is configured to be in contact with skin of a monitored object to obtain an electrocardiography signal of the monitored object, and transmit the electrocardiography signal to the processor. The processor is configured to process the electrocardiography signal. For effects achievable by the electronic device provided in the second aspect of this disclosure, refer to the effects corresponding to the first aspect and various implementation solutions of the first aspect. Details are not described herein again.

According to a third aspect, an embodiment of this disclosure further provides a fabrication method for an ECG electrode based on the first aspect. A transition layer is sputtered on the outer surface of the electrode substrate. The doped graphite-like carbon film is sputtered on a surface, away from the electrode substrate, of the transition layer. The doped graphite-like carbon film includes the graphite-like carbon, and the first nano-crystals and the second nano-crystals that are embedded in the graphite-like carbon. The graphite-like carbon includes the carbon element. The graphite-like carbon has the amorphous carbon structure. The first nano-crystal includes the weak carbon-bonding element. The weak carbon-bonding element has the part that exists in the form of the metallic elementary substance. The first nano-crystal includes the columnar structure. The columnar structure extends in the thickness direction of the doped graphite-like carbon film. The second nano-crystal includes the strong carbon-bonding element. The strong carbon-bonding element bonds with the carbon element in the graphite-like carbon through the chemical bond. The second nano-crystals are distributed between adjacent and spaced columnar structures. In the foregoing technical solution, the first nano-crystal of the columnar structure is embedded in the amorphous carbon structure of the graphite-like carbon, to enhance the conductivity of the graphite-like carbon in the thickness direction of the graphite-like carbon. In addition, the second nano-crystal is introduced in the amorphous carbon structure of the graphite-like carbon and the columnar structure of the first nano-crystal, to enhance the strength and the wear resistance of the doped graphite-like carbon film. In addition, the electrode substrate may be plated in a physical vapor deposition (Physical Vapor Deposition, PVD for short) manner, and fabricating the doped graphite-like carbon film through PVD is easy to operate and control and convenient to promote and use.

With reference to the third aspect, in a first possible implementation of the third aspect, the weak carbon-bonding element includes one or more of the following materials: Cu, Ag, Au, and Al. The doped graphite-like carbon film includes a first surface facing the electrode substrate and a second surface opposite to the first surface. An atomic percentage of the weak carbon-bonding element gradually increases in a thickness direction from the first surface to the second surface. A first nano-crystal close to the first surface is of a cluster structure. A first nano-crystal close to the second surface is of a columnar structure. In the foregoing technical solution, a columnar nano-crystal including the weak carbon-bonding elements Cu, Ag, Au, and Al has excellent conductivity, and may form a longitudinal conductive channel in the graphite-like carbon, so that the conductivity of the graphite-like carbon in the thickness direction of the graphite-like carbon is enhanced, and high ECG signal quality is achieved.

With reference to the first possible implementation of the third aspect, in a second possible implementation of the third aspect, the strong carbon-bonding element includes one or more of the following materials: Cr, W, and Ti. The doped graphite-like carbon film includes the first surface facing the electrode substrate and the second surface opposite to the first surface. An atomic percentage of the strong carbon-bonding element gradually decreases in the thickness direction from the first surface to the second surface. The second nano-crystals are distributed between a plurality of cluster structures and a plurality of columnar structures. In the foregoing technical solution, the strong carbon-bonding elements Cr, W, and Ti bond with the carbon element in the graphite-like carbon through chemical bonds, to generate second nano-crystals CrC, WC, and TiC. The foregoing three carbides are all hard carbides, and have advantages of high hardness and high wear resistance. The second nano-crystal is embedded in the amorphous carbon structure of the graphite-like carbon, to improve the hardness of the doped graphite-like carbon film and enhance the wear resistance of the doped graphite-like carbon film. In addition, the atomic percentage of the strong carbon-bonding element gradually decreases in the thickness direction from the first surface to the second surface. In this way, a great atomic percentage of the weak carbon-bonding element may be maintained close to the second surface, so that the doped graphite-like carbon film maintains high electrical signal quality close to the second surface. In addition, from the first surface to the second surface, the atomic percentage of the strong carbon-bonding element gradually decreases, and the atomic percentage of the weak carbon-bonding element gradually increases. Such a complementary design helps improve bonding force of the doped graphite-like carbon film.

With reference to the third aspect or any one of the first to the second possible implementations of the third aspect, in a third possible implementation of the third aspect, the transition layer includes one or more of the following materials: Cr, W, and Ti. In the foregoing technical solution, atoms may be diffused between the transition layer and the electrode substrate and between the transition layer and the doped graphite-like carbon film, to improve bonding force between the electrode substrate and the doped graphite-like carbon film.

With reference to the third aspect or any one of the first to the third possible implementations of the third aspect, in a fourth possible implementation of the third aspect, a thickness of the transition layer is greater than or equal to 0.2 µm and less than or equal to 0.6 µm, and a thickness of the doped graphite-like carbon film is greater than or equal to 1.0 µm and less than or equal to 1.4 µm. In the foregoing technical solution, the thicknesses of the transition layer and the doped graphite-like carbon film are moderate, so that the ECG electrode may have a longer service life while ensuring high electrocardiography signal quality. Therefore, user experience is enhanced.

### BRIEF DESCRIPTION OF DRAWINGS

To describe the technical solutions in examples of this disclosure or the conventional technology more clearly, the following briefly describes examples. The accompanying drawings in the following description show some examples of this disclosure, and persons skilled in the art may derive other drawings from these accompanying drawings without creative efforts.
FIG. 1 is a schematic diagram of a structure of an electronic device 100 according to an example of this disclosure;
FIG. 2 is a schematic diagram of a structure of an ECG electrode 20 according to an example of this disclosure; and
FIG. 3 is a flowchart of a fabrication process of an ECG electrode 20 according to an example of this disclosure.

### DETAILED DESCRIPTION

To make objectives, technical solutions, and effects of examples of this disclosure clearer, the following clearly and completely describes the technical solutions in examples of this disclosure with reference to specific examples. Apparently, the described examples are some but not all examples of this disclosure. All other examples obtained by persons skilled in the art based on examples of this disclosure without creative efforts shall fall within the protection scope of this disclosure.

The following describes specific examples of this disclosure with reference to the accompanying drawings.

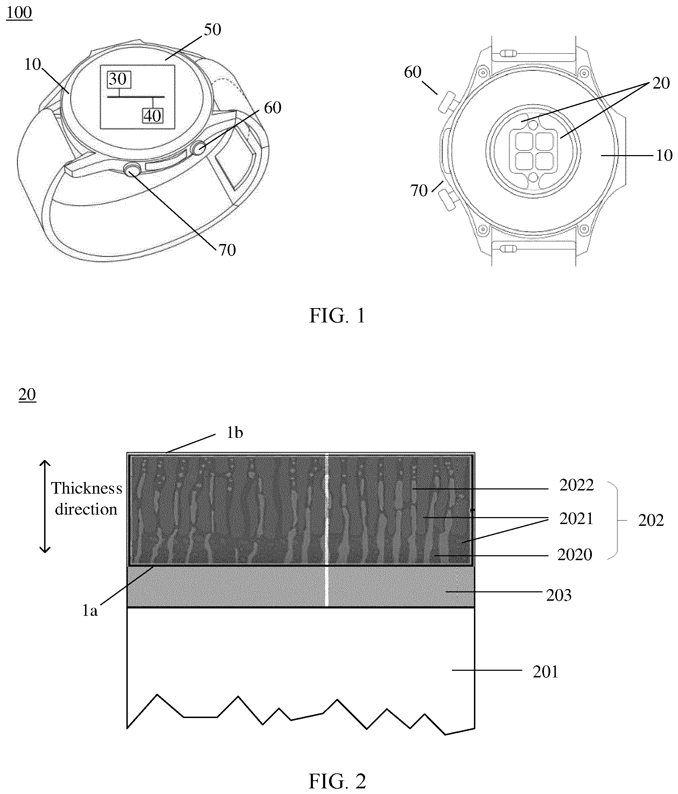

FIG. 1 is a schematic diagram of a structure of an electronic device 100 according to an example of this disclosure. The electronic device 100 has an electrocardiographic monitoring function. Specifically, the electronic device 100 may be an electronic product such as a mobile phone, a palmtop computer (personal digital assistant, PDA), a notebook computer, or a wearable device. The wearable device may be a smartwatch, a smart band, smart glasses, a headset, or the like. In this example of this disclosure, an example in which the electronic device 100 is a smartwatch is used for description.

In some examples, the electronic device 100 includes a housing 10, an ECG electrode 20, and a processor 30. The housing 10 is configured to accommodate and protect internal components (a movement, a watch face, a watch hand, and the like) of the electronic device 100. The housing 10 may be a rear housing of the electronic device 100, that is, a part of a housing that is in contact with skin of a user when the user wears the electronic device 100, or may be a complete housing. This is not limited in this example of this disclosure. A material of the housing 10 may be insulating ceramic, for example, zirconium dioxide (zirconium dioxide, ZrO₂) ceramic. The ECG electrode 20 may be embedded in a location that is on the housing 10 and that is in contact with the skin of the user. For example, the ECG electrode 20 may be located on the rear housing of the electronic device 100. When the user wears the electronic device 100, the ECG electrode 20 is in contact with the skin of the user. The ECG electrode 20 is configured to be in contact with skin of a monitored object to collect an electrocardiography signal of the monitored object. In this example of this disclosure, the ECG electrode 20 may be in a ring shape, a multi-segment ring shape (for example, a two-segment ring shape or a four-segment ring shape), or the like. The ECG electrode 20 may alternatively be obtained through profiling design based on a product shape, a wall thickness, and other features of the electronic device 100. The processor 30 is mounted to an inner side of the housing 10. The processor 30 is in communication connection to the ECG electrode 20. The processor 30 is configured to obtain the electrocardiography signal from the ECG electrode 20, and process the electrocardiography signal, specifically, for example, perform filtering, amplification, and analog-to-digital conversion on the collected electrocardiography signal, to finally generate electrocardiographic data.

In some examples, the electronic device 100 further includes a memory 40. The memory 40 is located inside the housing 10, and is in communication connection to the processor 30. The memory 40 is configured to store a software program or code. The processor 30 processes the electrocardiography signal by executing the software program or the code.

In some examples, the electronic device 100 further includes a display screen 50. The display screen 50 is fastened to the housing 10. The display screen 50 is electrically connected to the processor 30, and is configured to display the electrocardiographic data obtained through processing. The user may learn of the electrocardiographic data of the user by viewing a pattern (for example, an electrocardiographic waveform) displayed on the display screen 50. The display screen 50 may be a touch display screen.

In some examples, the electronic device 100 may further include a crown 60 and a button 70. The crown 60 and the button 70 may be disposed on a front-housing component 10. The front-housing component 10 is included in the housing 10. Both the crown 60 and the button 70 may perform some functions in response to an operation of the user. For example, the crown 60 is configured for date and time adjustment and winding. The button 70 may display a home screen in response to a pressing operation performed by the user with a finger. For example, the ECG electrode 20 may alternatively be located on an outer side of the crown 60 or an outer side of the button 70. The user may contact the crown 60 or the button 70 with a finger or another body part, so that the ECG electrode 20 collects the electrocardiography signal.

In this example, because the electronic device 100 is small and convenient to carry, the user may wear the electronic device 100 at any time to measure real-time electrocardiographic data of the user in time by using the ECG electrode 20, to detect a health status in time.

FIG. 2 is a schematic diagram of a film layer structure of the ECG electrode 20. In some examples, the ECG electrode 20 includes an electrode substrate 201 and a conductive film layer formed on the electrode substrate 201. When the electronic device 100 is used, the conductive film layer is close to the skin of the user relative to the electrode substrate 201. The ECG electrode 20 not only needs to have conductivity, but also needs to have high hardness and high wear resistance, to meet use performance of the electronic device 100 in electrocardiographic monitoring.

An example of this disclosure provides an ECG electrode 20. The ECG electrode 20 includes an electrode substrate 201, and further includes a doped graphite-like carbon film 202 located on an outer surface of the electrode substrate 201. The doped graphite-like carbon film 202 includes graphite-like carbon 2020, and first nano-crystals 2021 and second nano-crystals 2022 that are embedded in the graphite-like carbon 2020. In addition, the doped graphite-like carbon film 202 includes a first surface 1a facing the electrode substrate 201 and a second surface 1b opposite to the first surface 1a. Planes on which the first surface 1a and the second surface 1b are located are both approximately perpendicular to a thickness direction of the doped graphite-like carbon film 202.

**In** an example, the electrode substrate 201 may be made of stainless steel, titanium alloy, aluminum alloy, or tungsten steel. The electrode substrate 201 has good conductivity and a light weight, which is conducive to transmitting an electrical signal and may implement lightweight products. The graphite-like carbon (graphite-like carbon, GLC for short) 2020 includes a carbon element. The graphite-like carbon 2020 has an amorphous carbon structure. The amorphous carbon structure is a structure in which carbon atoms in the graphite-like carbon 2020 are not arranged regularly and periodically, the carbon atoms are arranged irregularly in general, but adjacent carbon atoms are arranged regularly. The graphite-like carbon 2020 has advantages of high hardness and good wear resistance, but has poor longitudinal conductivity. The longitudinal conductivity is conductivity in a thickness direction of the graphite-like carbon 2020. The thickness direction of the graphite-like carbon 2020 is equivalent to the thickness direction of the doped graphite-like carbon film 202. The graphite-like carbon 2020 has poor longitudinal conductivity because the amorphous carbon structure of the graphite-like carbon 2020 includes a large quantity of laminated planar structures, and the laminated planar structures are parallel to each other and are perpendicular to the thickness direction of the graphite-like carbon 2020. Because electrons cannot freely move between layers, resistivity in a direction perpendicular to the laminated planar structure, that is, in the thickness direction of the graphite-like carbon 2020, is high, which is reflected as poor conductivity of the graphite-like carbon 2020 in the thickness direction of the graphite-like carbon 2020.

In an example, the first nano-crystal 2021 is embedded in the amorphous carbon structure of the graphite-like carbon 2020, so that the conductivity of the graphite-like carbon 2020 in the thickness direction of the graphite-like carbon 2020 may be enhanced. Specifically, the first nano-crystal 2021 includes a weak carbon-bonding element. The weak carbon-bonding element is an element that has weak affinity with the carbon element and that is difficult to chemically bond with the carbon element to form a carbide. For example, the weak carbon-bonding element may be Cu, Ag, Au, or Al. The first nano-crystal 2021 includes at least one weak carbon-bonding element, that is, the first nano-crystal 2021 may include only the weak carbon-bonding element Cu, Ag, Au, or Al, or may include any combination of two or more of the foregoing weak carbon-bonding elements. The weak carbon-bonding element is difficult to chemically bond with the carbon element, and mainly exists in a form of a metallic elementary substance in the amorphous carbon structure. The metals Cu, Ag, Au, and Al have high conductivity, so that the formed first nano-crystal 2021 is embedded in the amorphous carbon structure of the graphite-like carbon 2020, to enhance the conductivity of the graphite-like carbon 2020.

Further, an atomic percentage of the weak carbon-bonding element may be controlled to gradually increase in a thickness direction from the first surface 1a to the second surface 1b, to enhance the conductivity of the graphite-like carbon 2020 in the thickness direction of the graphite-like carbon 2020. As the atomic percentage of the weak carbon-bonding element increases, the first nano-crystal 2021 is precipitated in a columnar structure. The columnar structure extends from the first surface 1a to the second surface 1b. The first nano-crystal 2021 of the columnar structure is also referred to as a columnar nano-crystal. The columnar nano-crystal including the weak carbon-bonding elements Cu, Ag, Au, and Al has excellent conductivity, and may form a longitudinal conductive channel in the graphite-like carbon 2020, so that the conductivity of the graphite-like carbon 2020 in the thickness direction of the graphite-like carbon 2020 is enhanced, and high ECG signal quality is achieved.

An atomic percentage of a weak carbon-bonding element close to the first surface 1a is low, and a first nano-crystal 2021 formed from the weak carbon-bonding element is precipitated in a cluster structure. An atomic percentage of a weak carbon-bonding element closer to the second surface 1b is greater, and a first nano-crystal 2021 formed from the weak carbon-bonding element is precipitated in a columnar structure. The atomic percentage of the weak carbon-bonding element is a ratio of a quantity of atoms of the weak carbon-bonding element to a total quantity of atoms in the doped graphite-like carbon film 202. The total quantity of atoms is a sum of a quantity of carbon atoms, the quantity of atoms of the weak carbon-bonding element, and a quantity of atoms of all other elements.

The first nano-crystal 2021 is embedded in the amorphous carbon structure, so that the graphite-like carbon 2020 may be conductive in the thickness direction of the graphite-like carbon 2020. However, introduction of the first nano-crystal 2021 reduces density inside the graphite-like carbon 2020, so that the hardness and the wear resistance of the graphite-like carbon 2020 are reduced. The second nano-crystal 2022 may be introduced into the graphite-like carbon 2020, to ensure strength and wear resistance of the graphite-like carbon 2020.

In an example, the second nano-crystal 2022 is embedded in the amorphous carbon structure of the graphite-like carbon 2020, so that the hardness and the wear resistance of the graphite-like carbon 2020 may be enhanced. Specifically, the second nano-crystal 2022 includes a strong carbon-bonding element. The strong carbon-bonding element is an element that has strong affinity with the carbon element and that easily chemically bonds with the carbon element to form a carbide. For example, the strong carbon-bonding element may be Cr, W, or Ti. The second nano-crystal 2022 includes at least one strong carbon-bonding element, that is, the second nano-crystal 2022 may include only the strong carbon-bonding element Cr, W, or Ti, or may include any combination of two or more of the foregoing strong carbon-bonding elements. The strong carbon-bonding element bonds with the carbon element in the graphite-like carbon 2020 through the chemical bond, to generate second nano-crystals CrC, WC, and TiC. The foregoing three carbides are all hard carbides, and have advantages of high hardness and high wear resistance. The second nano-crystal 2022 is embedded in the amorphous carbon structure of the graphite-like carbon 2020, to improve the hardness of the doped graphite-like carbon film 202 and enhance the wear resistance of the doped graphite-like carbon film 202.

In an embodiment, an atomic percentage of the strong carbon-bonding element gradually decreases in the thickness direction from the first surface 1a to the second surface 1b. This design may maintain a great atomic percentage of the weak carbon-bonding element close to the second surface 1b, so that the doped graphite-like carbon film 202 maintains high electrical signal quality close to the second surface 1b. In addition, from the first surface 1a to the second surface 1b, the atomic percentage of the strong carbon-bonding element gradually decreases, and the atomic percentage of the weak carbon-bonding element gradually increases. Such a complementary design helps improve bonding force of the doped graphite-like carbon film 202. The atomic percentage of the strong carbon-bonding element is a ratio of a quantity of atoms of the strong carbon-bonding element to a total quantity of atoms in the doped graphite-like carbon film 202. The total quantity of atoms is a sum of a quantity of carbon atoms, the quantity of atoms of the strong carbon-bonding element, and a quantity of atoms of all other elements.

In an example, the second nano-crystals 2022 are distributed between cluster structures and columnar structures of a plurality of first nano-crystals 2021. A distribution manner of the second nano-crystals 2022 and the first nano-crystals 2021 helps enhance bonding force inside the doped graphite-like carbon film 202. In addition, because the second nano-crystal 2022 is a hard carbide, embedding the second nano-crystals 2022 between the cluster structures and the columnar structures of the first nano-crystals 2021 helps better enhance the hardness of the entire doped graphite-like carbon film 202.

In conclusion, based on the doped graphite-like carbon film 202 including the graphite-like carbon 2020, and the first nano-crystals 2021 and the second nano-crystals 2022 that are both embedded in the graphite-like carbon 2020, not only the conductivity of the doped graphite-like carbon film 202 in the thickness direction of the doped graphite-like carbon film 202 but also high hardness and high wear resistance of the entire doped graphite-like carbon film 202 may be ensured. In this way, the ECG electrode 20 may have a longer service life while ensuring high electrocardiography signal quality. Therefore, user experience is enhanced.

In an example, the ECG electrode 20 further includes a transition layer 203. The transition layer 203 is located between the outer surface of the electrode substrate 201 and the doped graphite-like carbon film 202. Because the electrode substrate 201 and the doped graphite-like carbon film 202 are made of different materials, bonding force between the electrode substrate 201 and the doped graphite-like carbon film 202 is weak. Atoms may be diffused between the transition layer 203 and the electrode substrate 201 and between the transition layer 203 and the doped graphite-like carbon film 202, to improve the bonding force between the electrode substrate 201 and the doped graphite-like carbon film 202. Further, to enhance attachment effects of the doped graphite-like carbon film 202, the transition layer 203 is usually made of a same material as the strong carbon-bonding element, that is, the transition layer 203 includes one or more of the following materials: Cr, W, and Ti. For example, the transition layer 203 may be attached to the outer surface of the electrode substrate 201 in a plating manner such as magnetron sputtering, arc plasma plating, and ion plating. The foregoing plating manner is easy to operate and control and convenient to promote and use.

An example of this disclosure further provides a fabrication method for an ECG electrode 20. The ECG electrode 20 includes an electrode substrate 201 and a doped graphite-like carbon film 202 located on an outer surface of the electrode substrate 201. The doped graphite-like carbon film 202 includes graphite-like carbon 2020, and first nano-crystals 2021 and second nano-crystals 2022 that are doped in the graphite-like carbon 2020. The graphite-like carbon 2020 includes a carbon element. The graphite-like carbon 2020 has an amorphous carbon structure. The first nano-crystal 2021 includes a weak carbon-bonding element. The weak carbon-bonding element has a part that exists in a form of a metallic elementary substance. The first nano-crystal 2021 includes a columnar structure. The columnar structure extends in a thickness direction of the doped graphite-like carbon film 202. The second nano-crystal 2022 includes a strong carbon-bonding element. The strong carbon-bonding element bonds with the carbon element in the graphite-like carbon 2020 through a chemical bond. The second nano-crystals 2022 are distributed between adjacent and spaced columnar structures.

In an example, the electrode substrate 201 may be plated in a physical vapor deposition (English: Physical Vapor Deposition, PVD for short) manner. A PVD plating process includes: bombarding a target material by using a high-energy particle, so that atoms in the target material are sputtered out and then deposited on the outer surface of the electrode substrate 201 to form a thin film. The target material is a material with which the outer surface of the electrode substrate 201 is plated. In this example of this disclosure, the target material includes high-purity graphite, the weak carbon-bonding element, or the strong carbon-bonding element, and is used to form the graphite-like carbon 2020, the first nano-crystal 2021, or the second nano-crystal 2022.

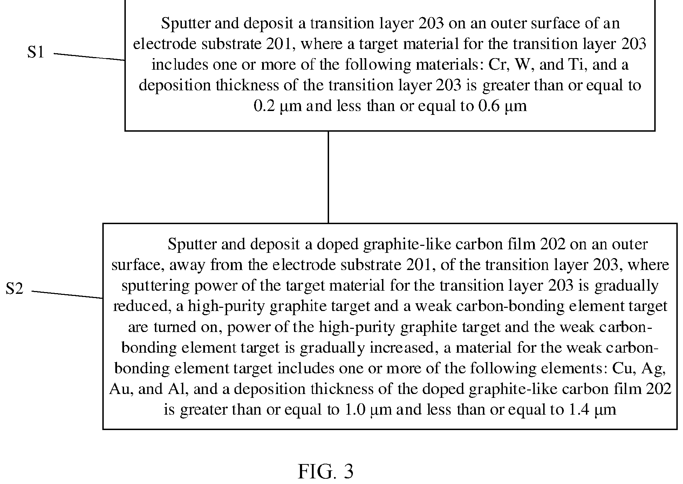

FIG. 3 is a flowchart of the fabrication method for the ECG electrode 20 according to an example of this disclosure. The fabrication method specifically includes the following steps.

Step S1: Sputter and deposit a transition layer 203 on the outer surface of the electrode substrate 201. The target material for the transition layer 203, that is, the strong carbon-bonding element, includes one or more of the following materials: Cr, W, and Ti. A deposition thickness of the transition layer 203 is greater than or equal to 0.2 µm and less than or equal to 0.6 µm.

Step S2: Sputter and deposit the doped graphite-like carbon film 202 on an outer surface, away from the electrode substrate 201, of the transition layer 203. After the transition layer 203 is deposited in step S1, sputtering power of the target material for the transition layer 203 is gradually reduced, a high-purity graphite target and a weak carbon-bonding element target are turned on, and power of the high-purity graphite target and the weak carbon-bonding element target is gradually increased. A material for the weak carbon-bonding element target includes one or more of the following elements: Cu, Ag, Au, and Al. A deposition thickness of the doped graphite-like carbon film 202 is greater than or equal to 1.0 µm and less than or equal to 1.4 µm.

In an example, the fabrication method for the ECG electrode 20 includes:
first, performing dewaxing and deoiling treatment on a surface of the electrode substrate 201 through ultrasonic cleaning, and then performing cleaning and drying by using propane and alcohol in sequence to obtain a clean surface of the electrode substrate 201;
then, controlling pressure inside a vacuum chamber to 0.4 Pa, and performing pre-sputtering cleaning on the clean surface of the electrode substrate 201 by using argon plasma; and
after cleaning is completed, performing PVD plating treatment on the electrode substrate 201, where an example in which the transition layer 203 is made of Cr, the weak carbon-bonding element is Ag, and the strong carbon-bonding element is Cr is used. A Cr target is first turned on, sputtering power of the Cr target is adjusted to 30 to 65 W, and deposition is performed for 10 to 20 min to obtain a Cr transition layer 203 with a thickness greater than or equal to 0.2 µm and less than or equal to 0.6 µm. After the transition layer 203 is deposited, the power of the Cr target is gradually reduced from 30 W to 65 W to 10 W to 35 W, and an Ag target and the high-purity graphite target are turned on. Sputtering power of the Ag target is gradually increased from 20 W to 70 W to 85 W to 150 W, and sputtering power of the high-purity graphite target is gradually increased from 150 W to 250 W to 350 W to 400 W. Finally, the doped graphite-like carbon film 202 with a thickness greater than or equal to 1.0 µm and less than or equal to 1.4 µm is obtained. Table 1 shows a plurality of examples of the doped graphite-like carbon film 202 obtained by changing element types or a quantity of element types of the weak carbon-bonding element, the strong carbon-bonding element, and the transition layer 203, and test data of the doped graphite-like carbon film 202. The test data includes a direct current offset voltage, composite offset instability and internal noise, hardness, and a wear rate.

It should be noted that, in this example of this disclosure, for a test of electrical signal quality of the ECG electrode 20, refer to the national standard YY/T 0196-2005 Disposable Electrocardiography Electrodes. A test instrument is an electrocardiography electrode performance tester YICE0196, which uses a test circuit that complies with the standard YY/T 0196-2005. In the test, an ambient temperature is 23±5°C, and a relative humidity is 40%±10%. In this test, the direct current offset voltage and the composite offset instability and internal noise are selected as indexes for measuring ECG signal quality of a plated metal electrode. For each type of electrodes, at least 25 pairs are tested. A test method is specifically as follows.

For the direct current offset voltage, a loop including two glue-to-glue electrodes and a direct current voltmeter with minimum input impedance of 10 MΩ and resolution of 1 mV or higher is used for measurement. According to performance requirements on electrodes in the national standard, the direct current offset voltage should not be greater than 100 mV after a 1-min stable phase.

For the composite offset instability and internal noise, according to the performance requirements on electrodes in the national standard, a voltage generated in a frequency band of 0.15 Hz to 100 Hz (first-order frequency response) after the 1-min stable phase should not exceed 150 µV (peak-to-peak) within the following 5 min.

In addition, a method for testing hardness and wear resistance of the doped graphite-like carbon film 202 in the plurality of examples is as follows.

The hardness is measured by using a nanoindenter.

For the wear rate, a profile of a wear scar section is measured and plotted by using a step profiler, and a specific wear rate W is calculated: W=(A×d)/(P×L), where A indicates an area of the wear scar section, d indicates a reciprocating sliding distance, P indicates a normal load, and L indicates a total stroke in a friction process.

**Table 1**

| Doped graphite-like carbon film | Film layer structure (transition layer/weak carbon-bonding element: strong carbon-bonding element: graphite-like carbon) | Hardness (GPa) | Wear rate (10⁻¹⁶·mm3·N-1m⁻¹) | Direct current offset voltage (mV) | Composit e offset instability and internal noise (µV) |
|---|---|---|---|---|---|
| First | Cr/Ag:Cr:GLC | 15 | 2.36 | ≤40 | ≤5 |
| Second | Cr/Cu:Cr:GLC | 12 | 3.98 | ≤83 | ≤130 |
| Third | Cr/Au:Cr:GLC | 11 | 8.88 | ≤30 | ≤5 |
| Fourth | Cr/Al:Cr:GLC | 16 | 7.59 | ≤98 | ≤95 |
| Fifth | Cr/W/Ag:W:GLC | 21 | 1.21 | ≤15 | ≤10 |
| Sixth | Cr/W/Cu:W:GLC | 17 | 1.36 | ≤38 | ≤120 |
| Seventh | Cr/W/Au:W:GLC | 18 | 1.78 | ≤95 | ≤12 |
| Eighth | Cr/W/Al:W:GLC | 18 | 3.65 | ≤70 | ≤136 |
| Ninth | Cr/Ti/Ag:Ti:GLC | 24 | 2.11 | ≤56 | ≤15 |
| Tenth | Cr/Ti/Cu:Ti:GLC | 22 | 3.21 | ≤89 | ≤10 |
| Eleventh | Cr/Ti/Au: Ti: GLC | 18 | 6.33 | ≤23 | ≤16 |
| Twelfth | Cr/Ti/Al:Ti:GLC | 19 | 5.48 | ≤80 | ≤79 |
| Thirteenth | Cr/Ag:Au:Cr:GLC | 11 | 18.6 | ≤15 | ≤10 |
| Fourteenth | Cr/Ag:Cu:Cr:GLC | 9 | 23.3 | ≤60 | ≤70 |
| Fifteenth | Cr/Au:Cu:Cr:GLC | 10 | 22.5 | ≤66 | ≤130 |
| Sixteenth | Cr/GLC | 12 | 12.8 | ≤450 | ≤300 |
| Seventeenth | Cr/Cr:GLC | 17 | 9.63 | ≤300 | ≤180 |
| Eighteenth | Cr/Ag:GLC | 4 | 85.7 | ≤60 | ≤5 |

It may be learned from the data in Table 1 that, compared with the sixteenth and the seventeenth doped graphite-like carbon films without the weak carbon-bonding element, the direct current offset voltage and the composite offset instability and internal noise are significantly reduced in the first to the fifteenth doped graphite-like carbon films with the weak carbon-bonding element, which indicates that the first nano-crystal 2021 formed from the weak carbon-bonding element has significant effects on improving the conductivity of the graphite-like carbon 2020. Compared with the eighteenth doped graphite-like carbon film without the strong carbon-bonding element, the first to the fifteenth doped graphite-like carbon films have higher hardness and lower wear rates, which indicates that the second nano-crystal 2022 formed from the strong carbon-bonding element has significant effects on improving the hardness and the wear resistance of the doped graphite-like carbon film 202.

According to the fabrication method for the ECG electrode provided in this example of this disclosure, as an atomic percentage of the weak carbon-bonding element in the doped graphite-like carbon film increases, the first nano-crystal formed from the weak carbon-bonding element is first precipitated in a cluster structure until the first nano-crystal of the columnar structure that penetrates the doped graphite-like carbon film is generated, so that a stable longitudinal conductive channel may be formed in the doped graphite-like carbon film, to enhance conductivity of the doped graphite-like carbon film in the thickness direction of the doped graphite-like carbon film. **In** addition, the strong carbon-bonding element chemically bonds with the carbon element in the graphite-like carbon to generate the second nano-crystal, and the second nano-crystal is embedded between the graphite-like carbon and the first nano-crystal, to improve density and hardness of the doped graphite-like carbon film and enhance wear resistance. Therefore, embedding the first nano-crystal and the second nano-crystal in the graphite-like carbon may enhance the conductivity, the hardness, and the wear resistance of the graphite-like carbon. Therefore, an ECG electrode with both excellent ECG signal quality and high hardness and wear resistance is obtained.

## Claims

1. An electrocardiography, ECG, electrode (20), comprising an electrode substrate (201) and a doped graphite-like carbon film (202) located on an outer surface of the electrode substrate, wherein the doped graphite-like carbon film comprises graphite-like carbon (2020), and first nano-crystal (2021) and second nano-crystals (2022) that are doped in the graphite-like carbon, the graphite-like carbon comprises a carbon element, and the graphite-like carbon has an amorphous carbon structure;
the first nano-crystal comprises a weak carbon-bonding element, the weak carbon-bonding element has a part that exists in a form of a metallic elementary substance, the first nano-crystal comprises a columnar structure, and the columnar structure extends in a thickness direction of the doped graphite-like carbon film;
the second nano-crystal comprises a strong carbon-bonding element, and the strong carbon-bonding element bonds with the carbon element in the graphite-like carbon through a chemical bond; and
the second nano-crystals are distributed between adjacent and spaced columnar structures, wherein the doped graphite-like carbon film comprises a first surface (1a) facing the electrode substrate and a second surface (1b) opposite to the first surface, and **characterised in that**
an atomic percentage of the weak carbon-bonding element gradually increases in a thickness direction from the first surface to the second surface, and/or
an atomic percentage of the strong carbon-bonding element gradually decreases in the thickness direction from the first surface to the second surface.

2. The ECG electrode according to claim 1, wherein
the weak carbon-bonding element comprises one or more of the following materials: Cu, Ag, Au, and Al.

3. The ECG electrode according to any one of claims 1 to 2, wherein
a first nano-crystal close to the first surface is of a cluster structure, and a first nano-crystal close to the second surface is of a columnar structure.

4. The ECG electrode according to claim 3, wherein
the second nano-crystals are distributed between a plurality of cluster structures and a plurality of columnar structures.

5. The ECG electrode according to any one of claims 1 to 4, wherein
the strong carbon-bonding element comprises one or more of the following materials: Cr, W, and Ti.

6. The ECG electrode according to any one of claims 1 to 5, wherein the ECG electrode further comprises a transition layer (203), and the transition layer is located between the electrode substrate and the doped graphite-like carbon film.

7. The ECG electrode according to claim 6, wherein
the transition layer comprises one or more of the following materials: Cr, W, and Ti.

8. An electronic device (100), comprising a processor (30) and the ECG electrode according to any one of claims 1 to 7, wherein the ECG electrode is configured to be in contact with skin of a monitored object to obtain an electrocardiography signal of the monitored object, and transmit the electrocardiography signal to the processor; and the processor is configured to process the electrocardiography signal.

9. A method for manufacturing an electrocardiography, ECG, electrode (20), comprising:
sputtering (S1) a transition layer (203) on an outer surface of an electrode substrate (201); and
sputtering (S2) a doped graphite-like carbon film (202) on a surface of the transition layer, away from the electrode substrate, wherein the doped graphite-like carbon film comprises graphite-like carbon (2020) and first nano-crystals (2021) and second nano-crystals (2022) that are embedded in the graphite-like carbon, the graphite-like carbon comprises a carbon element, and the graphite-like carbon has an amorphous carbon structure; the first nano-crystal comprises a weak carbon-bonding element, the weak carbon-bonding element has a part that exists in a form of a metallic elementary substance, the first nano-crystal comprises a columnar structure, and the columnar structure extends in a thickness direction of the doped graphite-like carbon film; the second nano-crystal comprises a strong carbon-bonding element, and the strong carbon-bonding element bonds with the carbon element in the graphite-like carbon through a chemical bond; and the second nano-crystals are distributed between adjacent and spaced columnar structures,
wherein the doped graphite-like carbon film comprises a first surface (1a) facing the electrode substrate and a second surface (1b) opposite to the first surface, and wherein
an atomic percentage of the weak carbon-bonding element gradually increases in a thickness direction from the first surface to the second surface, and/or
an atomic percentage of the strong carbon-bonding element gradually decreases in the thickness direction from the first surface to the second surface.

10. The method according to claim 9, wherein
the weak carbon-bonding element comprises one or more of the following materials: Cu, Ag, Au, and Al.

11. The method according to claim 9 or 10, wherein
a first nano-crystal close to the first surface is of a cluster structure, and a first nano-crystal close to the second surface is of a columnar structure.

12. The method according to any of claims 9 to 11, wherein
the strong carbon-bonding element comprises one or more of the following materials: Cr, W, and Ti.

13. The method according to any of claims 9 to 12, wherein
the second nano-crystals are distributed between a plurality of cluster structures and a plurality of columnar structures.

14. The method according to any one of claims 9 to 13, wherein
the transition layer comprises one or more of the following materials: Cr, W, and Ti.

15. The method according to any one of claims 9 to 14, wherein
a thickness of the transition layer is greater than or equal to 0.2 µm and less than or equal to 0.6 µm, and a thickness of the doped graphite-like carbon film is greater than or equal to 1.0 µm and less than or equal to 1.4 µm.

## Patentansprüche

1. Elektrokardiographie-Elektrode, EKG-Elektrode (20), umfassend ein Elektrodensubstrat (201) und einen dotierten graphitähnlichen Kohlenstofffilm (202), der sich auf einer Außenfläche des Elektrodensubstrats befindet, wobei der dotierte graphitähnliche Kohlenstofffilm graphitähnlichen Kohlenstoff (2020) sowie einen ersten Nanokristall (2021) und zweite Nanokristalle (2022) umfasst, die in den graphitähnlichen Kohlenstoff dotiert sind, der graphitähnliche Kohlenstoff ein Kohlenstoffelement umfasst und der graphitähnliche Kohlenstoff eine amorphe Kohlenstoffstruktur aufweist;
der erste Nanokristall ein schwaches Kohlenstoffbindungselement umfasst, das schwache Kohlenstoffbindungselement einen Teil aufweist, der in einer Form einer metallischen Elementarsubstanz vorliegt, der erste Nanokristall eine säulenförmige Struktur umfasst und sich die säulenförmige Struktur in einer Dickenrichtung des dotierten graphitartigen Kohlenstofffilms erstreckt;
der zweite Nanokristall ein starkes Kohlenstoffbindungselement umfasst, und sich das starke Kohlenstoffbindungselement durch eine chemische Bindung mit dem Kohlenstoffelement in dem graphitähnlichen Kohlenstoff verbindet; und
die zweiten Nanokristalle zwischen benachbarten und beabstandeten säulenförmigen Strukturen verteilt sind,
wobei der dotierte graphitartige Kohlenstofffilm eine dem Elektrodensubstrat zugewandte erste Oberfläche (1a) und eine der ersten Oberfläche gegenüberliegende zweite Oberfläche (1b) umfasst, und **dadurch gekennzeichnet, dass**
ein Atomprozentsatz des schwachen Kohlenstoffbindungselements in einer Dickenrichtung von der ersten Oberfläche zu der zweiten Oberfläche graduell zunimmt, und/oder
ein Atomprozentsatz des starken Kohlenstoffbindungselements in der Dickenrichtung von der ersten Oberfläche zu der zweiten Oberfläche graduell abnimmt.

2. EKG-Elektrode nach Anspruch 1, wobei
das schwache Kohlenstoffbindungselement ein oder mehrere der folgenden Materialien umfasst: Cu, Ag, Au und Al.

3. EKG-Elektrode nach einem der Ansprüche 1 bis 2, wobei
ein erster Nanokristall in der Nähe der ersten Oberfläche eine Clusterstruktur aufweist, und ein erster Nanokristall in der Nähe der zweiten Oberfläche eine säulenförmige Struktur aufweist.

4. EKG-Elektrode nach Anspruch 3, wobei
die zweiten Nanokristalle zwischen einer Vielzahl von Clusterstrukturen und einer Vielzahl von säulenförmigen Strukturen verteilt sind.

5. EKG-Elektrode nach einem der Ansprüche 1 bis 4, wobei
das starke Kohlenstoffbindungselement ein oder mehrere der folgenden Materialien umfasst: Cr, W und Ti.

6. EKG-Elektrode nach einem der Ansprüche 1 bis 5, wobei
die EKG-Elektrode ferner eine Übergangsschicht (203) umfasst, und sich die Übergangsschicht zwischen dem Elektrodensubstrat und dem dotierten graphitartigen Kohlenstofffilm befindet.

7. EKG-Elektrode nach Anspruch 6, wobei
die Übergangsschicht ein oder mehrere der folgenden Materialien umfasst: Cr, W und Ti.

8. Elektronische Vorrichtung (100), umfassend einen Prozessor (30) und die EKG-Elektrode nach einem der Ansprüche 1 bis 7, wobei die EKG-Elektrode dazu konfiguriert ist, mit der Haut eines überwachten Objekts in Kontakt zu stehen, um ein Elektrokardiographiesignal des überwachten Objekts zu erlangen, und das Elektrokardiographiesignal an den Prozessor zu übertragen; und der Prozessor dazu konfiguriert ist, das Elektrokardiographiesignal zu verarbeiten.

9. Verfahren zur Herstellung einer Elektrokardiographie-Elektrode, EKG-Elektrode (20), umfassend:
Aufsprühen (S1) einer Übergangsschicht (203) auf eine Außenfläche eines Elektrodensubstrats (201); und
Aufsprühen (S2) eines dotierten graphitartigen Kohlenstofffilms (202) auf eine Oberfläche der Übergangsschicht, die von dem Elektrodensubstrat entfernt ist,
wobei der dotierte graphitähnliche Kohlenstofffilm graphitähnlichen Kohlenstoff (2020) sowie erste Nanokristalle (2021) und zweite Nanokristalle (2022) umfasst, die in den graphitähnlichen Kohlenstoff eingebettet sind, der graphitähnliche Kohlenstoff ein Kohlenstoffelement umfasst und der graphitähnliche Kohlenstoff eine amorphe Kohlenstoffstruktur aufweist; der erste Nanokristall ein schwaches Kohlenstoffbindungselement umfasst, das schwache Kohlenstoffbindungselement einen Teil aufweist, der in einer Form einer metallischen Elementsubstanz vorliegt, der erste Nanokristall eine säulenförmige Struktur umfasst, und sich die säulenförmige Struktur in einer Dickenrichtung des dotierten graphitähnlichen Kohlenstofffilms erstreckt; der zweite Nanokristall ein starkes Kohlenstoffbindungselement umfasst und sich das starke Kohlenstoffbindungselement über eine chemische Bindung mit dem Kohlenstoffelement in dem graphitähnlichen Kohlenstoff verbindet; und die zweiten Nanokristalle zwischen benachbarten und beabstandeten säulenförmigen Strukturen verteilt sind,
wobei der dotierte graphitartige Kohlenstofffilm eine dem Elektrodensubstrat zugewandte erste Oberfläche (1a) und eine der ersten Oberfläche gegenüberliegende zweite Oberfläche (1b) umfasst, und wobei
ein Atomprozentsatz des schwachen Kohlenstoffbindungselements in einer Dickenrichtung von der ersten Oberfläche zu der zweiten Oberfläche graduell zunimmt, und/oder
ein Atomprozentsatz des starken Kohlenstoffbindungselements in der Dickenrichtung von der ersten Oberfläche zu der zweiten Oberfläche graduell abnimmt.

10. Verfahren nach Anspruch 9, wobei
das schwache Kohlenstoffbindungselement ein oder mehrere der folgenden Materialien umfasst: Cu, Ag, Au und Al.

11. Verfahren nach Anspruch 9 oder 10, wobei
ein erster Nanokristall in der Nähe der ersten Oberfläche eine Clusterstruktur aufweist, und ein erster Nanokristall in der Nähe der zweiten Oberfläche eine säulenförmige Struktur aufweist.

12. Verfahren nach einem der Ansprüche 9 bis 11, wobei
das starke Kohlenstoffbindungselement ein oder mehrere der folgenden Materialien umfasst: Cr, W und Ti.

13. Verfahren nach einem der Ansprüche 9 bis 12, wobei
die zweiten Nanokristalle zwischen einer Vielzahl von Clusterstrukturen und einer Vielzahl von säulenförmigen Strukturen verteilt sind.

14. Verfahren nach einem der Ansprüche 9 bis 13, wobei
die Übergangsschicht ein oder mehrere der folgenden Materialien umfasst: Cr, W und Ti.

15. Verfahren nach einem der Ansprüche 9 bis 14, wobei
eine Dicke der Übergangsschicht größer oder gleich 0,2 µm und kleiner oder gleich 0,6 µm ist, und eine Dicke des dotierten graphitartigen Kohlenstofffilms größer oder gleich 1,0 µm und kleiner oder gleich 1,4 µm ist.

## Revendications

1. Électrode d'électrocardiographie, ECG (20), comprenant un substrat d'électrode (201) et un film de carbone de type graphite dopé (202) situé sur une surface extérieure du substrat d'électrode, dans laquelle le film de carbone de type graphite dopé comprend du carbone de type graphite (2020), et un premier nanocristal (2021) et des seconds nanocristaux (2022) qui sont dopés dans le carbone de type graphite, le carbone de type graphite comprend un élément carbone, et le carbone de type graphite présente une structure de carbone amorphe ;
le premier nanocristal comprend un élément de liaison carbone faible, l'élément de liaison carbone faible présente une partie qui existe sous la forme d'une substance élémentaire métallique, le premier nanocristal comprend une structure colonnaire, et la structure colonnaire se prolonge dans une direction d'épaisseur du film de carbone de type graphite dopé ;
le second nanocristal comprend un élément de liaison carbone fort, et l'élément de liaison carbone fort se lie à l'élément carbone dans le carbone de type graphite par une liaison chimique ; et
les seconds nanocristaux sont répartis entre des structures colonnaires adjacentes et espacées,
dans laquelle le film de carbone de type graphite dopé comprend une première surface (1a) faisant face au substrat d'électrode et une seconde surface (1b) opposée à la première surface, et **caractérisée en ce que**
un pourcentage atomique de l'élément de liaison carbone faible augmente progressivement dans une direction d'épaisseur de la première surface à la seconde surface, et/ou
un pourcentage atomique de l'élément de liaison carbone fort diminue progressivement dans la direction d'épaisseur de la première surface à la seconde surface.

2. Électrode ECG selon la revendication 1, dans laquelle l'élément de liaison carbone faible comprend un ou plusieurs des matériaux suivants : Cu, Ag, Au et Al.

3. Électrode ECG selon l'une quelconque des revendications 1 et 2, dans laquelle
un premier nanocristal proche de la première surface est d'une structure en cluster, et un premier nanocristal proche de la seconde surface est d'une structure colonnaire.

4. Électrode ECG selon la revendication 3, dans laquelle
les seconds nanocristaux sont répartis entre une pluralité de structures en cluster et une pluralité de structures colonnaires.

5. Électrode ECG selon l'une quelconque des revendications 1 à 4, dans laquelle
l'élément de liaison carbone fort comprend un ou plusieurs des matériaux suivants : Cr, W et Ti.

6. Électrode ECG selon l'une quelconque des revendications 1 à 5, dans laquelle
l'électrode ECG comprend également une couche de transition (203), et la couche de transition est située entre le substrat d'électrode et le film de carbone de type graphite dopé.

7. Électrode ECG selon la revendication 6, dans laquelle
la couche de transition comprend un ou plusieurs des matériaux suivants : Cr, W et Ti.

8. Dispositif électronique (100), comprenant un processeur (30) et l'électrode ECG selon l'une quelconque des revendications 1 à 7, dans lequel l'électrode ECG est configurée pour être en contact avec la peau d'un objet surveillé pour obtenir un signal d'électrocardiographie de l'objet surveillé, et transmettre le signal d'électrocardiographie au processeur ; et le processeur est configuré pour traiter le signal d'électrocardiographie.

9. Procédé de fabrication d'une électrode d'électrocardiographie, ECG (20), comprenant :
la pulvérisation (S1) d'une couche de transition (203) sur une surface extérieure d'un substrat d'électrode (201) ; et
la pulvérisation (S2) d'un film de carbone de type graphite dopé (202) sur une surface de la couche de transition, à l'écart du substrat d'électrode,
dans lequel le film de carbone de type graphite dopé comprend du carbone de type graphite (2020) et des premiers nanocristaux (2021) et des seconds nanocristaux (2022) noyés dans le carbone de type graphite, le carbone de type graphite comprend un élément carbone et le carbone de type graphite présente une structure de carbone amorphe ; le premier nanocristal comprend un élément de liaison carbone faible, l'élément de liaison carbone faible présente une partie qui se présente sous la forme d'une substance élémentaire métallique, le premier nanocristal comprend une structure colonnaire, et la structure colonnaire se prolonge dans une direction d'épaisseur du film de carbone de type graphite dopé ; le second nanocristal comprend un élément de liaison carbone fort et l'élément de liaison carbone fort se lie à l'élément carbone du carbone de type graphite à travers une liaison chimique ; et les seconds nanocristaux sont répartis entre des structures colonnaires adjacentes et espacées,
dans lequel le film de carbone de type graphite dopé comprend une première surface (1a) faisant face au substrat d'électrode et une seconde surface (1b) opposée à la première surface, et dans lequel
un pourcentage atomique de l'élément de liaison carbone faible augmente progressivement dans une direction d'épaisseur de la première surface à la seconde surface, et/ou
un pourcentage atomique de l'élément de liaison carbone fort diminue progressivement dans la direction d'épaisseur de la première surface à la seconde surface.

10. Procédé selon la revendication 9, dans lequel
l'élément de liaison carbone faible comprend un ou plusieurs des matériaux suivants : Cu, Ag, Au et Al.

11. Procédé selon la revendication 9 ou 10, dans lequel
un premier nanocristal proche de la première surface est d'une structure en cluster, et un premier nanocristal proche de la seconde surface est d'une structure colonnaire.

12. Procédé selon l'une quelconque des revendications 9 à 11, dans lequel
l'élément de liaison carbone fort comprend un ou plusieurs des matériaux suivants : Cr, W et Ti.

13. Procédé selon l'une quelconque des revendications 9 à 12, dans lequel
les seconds nanocristaux sont répartis entre une pluralité de structures en cluster et une pluralité de structures colonnaires.

14. Procédé selon l'une quelconque des revendications 9 à 13, dans lequel
la couche de transition comprend un ou plusieurs des matériaux suivants : Cr, W et Ti.

15. Procédé selon l'une quelconque des revendications 9 à 14, dans lequel
une épaisseur de la couche de transition est supérieure ou égale à 0,2 µm et inférieure ou égale à 0,6 µm, et une épaisseur du film de carbone de type graphite dopé est supérieure ou égale à 1,0 µm et inférieure ou égale à 1,4 µm.
